# EUROPEAN PATENT APPLICATION

(11) **EP 4 545 126 A1**
(43) Date of publication of application: **30.04.2025**
(21) Application number: 23827259.5
(22) Date of filing: 22.06.2023
(51) Int. Cl.: A61M 16/10, G10K 11/16

(54) **NOISE REDUCTION BOX**

(30) Priority: 24.06.2022 JP 2022101496
(71) Applicant: Teijin Pharma Limited, Tokyo 100-0013 (JP)
(72) Inventor: HIRAI, Yuichi, Tokyo 100-0013 (JP); ITO, Shinichi, Tokyo 100-0013 (JP)
(74) Representative: Hoffmann Eitle
(86) International application number: PCT/JP2023/023125
(87) International publication number: WO 2023/249077

(57) **Abstract**

To provide a noise reduction box equipped with a storage portion for storing a device inside of the opening/closing door, a box air intake port for taking outside air into the noise reduction box, a box exhaust port for exhausting air in the noise reduction box, and an exhaust duct in the housing for guiding exhaust air to the box exhaust port, wherein a device exhaust port of the device stored in the storage portion comes into close contact with an inlet of the exhaust duct when the opening/closing door is closed, and equipment solving the problems required for a portable device, such as small size and light weight, low noise, power saving, and exhaust heat at the same time.

## Description

### [Technical Field]

The present invention relates to a noise reduction box for reducing noise generated by a small medical device.

### [Background Art]

In addition to chronic respiratory diseases, the recent spread of coronaviruses has led to an increase in the use of oxygen inhalation therapy for patients with respiratory diseases, and the demand for medical oxygen concentrators is growing. In addition to the stationary oxygen concentrators at home for supplying high dose oxygen of 3 L/min or 5 L/min, there have been developed portable concentrators that are small, light-weight, and for long-time use, which enables use not only for going to hospital, but also for outdoors such as shopping and leisure activities, and thus contributes to improvement in QOL (Quality of Life) of patients.

Oxygen concentrators are used while sleeping at night as well as during the day, and thus are required for calmness of the device. Most of the oxygen concentrators adopt pressure swing adsorption-type using a nitrogen-selective adsorbent over oxygen under pressurization, and thus are equipped with power elements such as compressors and motors, and cooling fans, which constitute noise sources along with the intake and exhaust sounds during oxygen generation. For this reason, various measures are taken, such as installing silencers in the intake pipe for the feed air and in the exhaust pipe for discharging the adsorbed nitrogen, noise-reducing duct structures, and installing sound-absorbing members in the casing. For example, JP-A-7-255850 (PTL 1) discloses a soundproof box for an oxygen concentrator in which a duct structure with lamination of sound-absorbing material is provided in the ventilation path and exhaust path of the housing.

In contrast, portable oxygen concentrators have been developed with priority on easy-carrying by patients and using outdoors, specifically on small size, light weight, and power saving, over noise levels, resulting in a tendency of reducing the number of components for calmness of the device. Although the calmness of the compressor itself has improved owing to recent technological advances, the noise level of portable devices remains higher compared with that of stationary devices. There is also a demand for using two devices of a stationary one at home and an oxygen cylinder or portable one for going out, which however causes a problem of the increased cost burden of devices due to the expensive price. JP-A-7-136274 (PTL 2) discloses a portable box made of a material having sound insulation properties for housing a portable oxygen concentrator.

### [Citation List]

### [Patent Literature]

[PTL 1] JP-A-7-255850
[PTL 2] JP-A-7-136274

### [Summary of Invention]

### [Technical Problem]

A noise reduction box, when storing a portable oxygen concentrator to achieve a noise reduction effect, is required to exhibit airtightness for preventing sound leakage. In contrast, the portable oxygen concentrator is equipped with a motor and a compressor, and semiconductors for controlling them, which are also heat sources, and it is required to cool the inside of the device to maintain the nitrogen adsorption efficiency of the adsorbent. The noise reduction box described in PTL 2 is equipped with an exhaust fan solely to prevent a temperature rise inside the box.

The portable device has problem-solving means conflicting with each other: one for small size and light weight, and the others for low noise, power saving, and exhaust heat, and the above exhaust fan equipped to the noise reduction box requires a new power source, which also becomes a noise source. The present invention has found out a method for solving all the problems at the same time such as low noise, power saving, and exhaust heat, by reconsidering the method of arranging devices in a noise reduction box and using an exhaust duct in combination.

### [Solution to Problem]

The present invention provides a noise reduction box equipped with a storage portion for storing a noise-generating device inside; a housing of the noise reduction box; and on the housing, an opening/closing door, a box air intake port for taking outside air into the noise reduction box, and a box exhaust port for exhausting air in the noise reduction box; and inside the box, an exhaust duct for guiding exhaust air to the box exhaust port; wherein a device exhaust port of the device stored in the storage portion comes into close contact with an inlet of the exhaust duct by closing the opening/closing door. Such a noise reduction box exposes the storage portion when the opening/closing door is opened, and allows the device to be stored in the storage portion. The device to be stored is air-cooled and has a device air intake port and a device exhaust port, and closing the opening/closing door will move the storage portion and bring the device exhaust port into close contact with the exhaust duct, enabling the exhaust heat air after cooling to be directly exhausted outside the system.

The present invention also provides a noise reduction box equipped with the storage portion fastened to the opening/closing door and storing an air-cooled device incorporating an air-cooling fan and provided with an air intake port and exhaust port on the device casing; the opening/closing door, arranged at the side of the noise reduction box, having a rotating shaft arranged horizontally under the box, opening diagonally upward with the rotating shaft as a fulcrum by pulling the upper part of the side toward an operator; the storage portion inside the opening/closing door; and the exhaust duct; wherein the device exhaust port and the inlet of the exhaust duct come into close contact in conjunction with the action of closing the opened door.

The present invention also provides a noise reduction box for a portable oxygen concentrator, wherein the device is an adsorption-type oxygen concentrator.

The present invention also provides an oxygen supply system, equipped with an adsorption-type oxygen concentrator; and a noise reduction box equipped with a storage portion for storing the adsorption-type oxygen concentrator inside, a housing of the noise reduction box; and on the housing, an opening/closing door, a box air intake port for taking outside air into the noise reduction box, and a box exhaust port for exhausting air in the noise reduction box; and inside the box, an exhaust duct for guiding exhaust air to the box exhaust port; wherein an exhaust port of the adsorption-type oxygen concentrator stored in the storage portion is in close contact with an inlet of the exhaust duct when the opening/closing door is closed. Such an opening/closing door is provided at the side of the noise reduction box, is connected to the box body via a rotating shaft arranged horizontally under the noise reduction box, and when pulled toward an operator at the upper part, opens diagonally upward with the rotating shaft as a fulcrum, and when closed, allows the exhaust port of the adsorption-type oxygen concentrator stored from the diagonally upward opening into the storage portion to come into close contact with the inlet of the exhaust duct.

### [Advantageous Effects of Invention]

The noise reduction box of the present invention, when the opening/closing door is opened, moves the storage portion to an exposed position in the opening to facilitate the operation of storing the device, and when the door is closed, moves the storage portion to a position allowing the exhaust port of the device to be in close contact with the exhaust duct, thereby achieving compatibility between ensuring good storage operability and preventing a decrease in cooling efficiency due to air leakage between the exhaust port of the device and the exhaust duct. From the viewpoint of reducing noise, the device is preferably stored near the center of the noise reduction box, and also from that point of view, preferred is a mechanism that enables the storage portion to move to near the center of the box by the open/close operation of the door.

Further, adopted is a mechanism in which the opening/closing door is provided at the side of the noise reduction box, is connected to a rotating shaft arranged horizontally at the lower part of the noise reduction box and is opened/closed when pulled diagonally downward toward an operator; and thus fastening the storage portion to the inside of the door allows this small operating force of closing the door using the principle of leverage to bring the inlet of the exhaust duct provided inside and the exhaust port of the device into close contact, and the exhaust path to be connected without air leaks.

Further, fastening the storage portion to the inside of the opening/closing door enables realization of a structure to move the storage portion without requiring a complex mechanism. The opening/closing door and the storage portion may be formed as an integrally molded member, or may be joined together by a bonding material or a bonding material having a shock-absorbing mechanism. The provided shock-absorbing mechanism can prevent vibrations of the stored device from being transmitted to the door to cause a noise leak.

The cooling fan provided in the devices to be stored such as oxygen concentrator also serves as the cooling means for the noise reduction box, and such a structure enables prevention of temperature rise inside the noise reduction box without providing an exhaust heat means such as a new air-cooling fan inside the box. The exhaust duct also provides a noise reduction effect, and solves the problems of low noise, power saving, exhaust heat, and operability all at once.

### [Brief Description of Drawings]

Fig. 1 shows a schematic diagram of an oxygen concentrator.
Fig. 2 shows a perspective view of an embodiment of the noise reduction box of the present invention.
Fig. 3 shows a cross-sectional view of an embodiment of the noise reduction box of the present invention.
Fig. 4 shows a procedure for storing an oxygen concentrator in the noise reduction box of the present invention.

### [Description of Embodiments]

The noise reduction box of the present invention will be described taking an example of an oxygen concentrator as the device stored inside the box for the purpose of reducing noise.

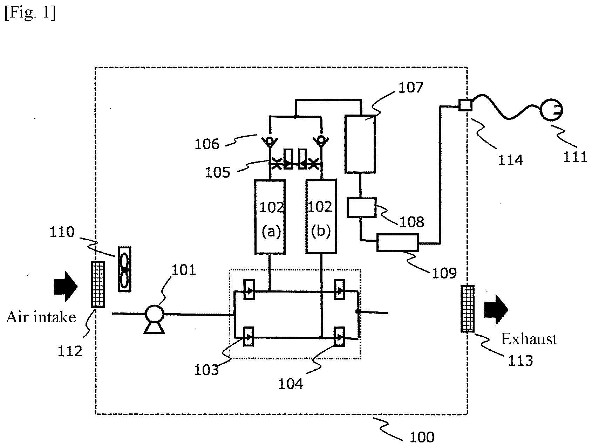

Fig. 1 shows a schematic diagram of an adsorption-type oxygen concentrator, which is one of the devices to be stored in the noise reduction box of the present invention. Adsorption-type oxygen concentrator 100 is equipped with compressor 101 for supplying feed air, adsorption cylinder 102 filled with an adsorbent selectively adsorbing nitrogen over oxygen, supply valve 103 of a flow path switching means for switching adsorption/desorption step, exhaust valve 104, and pressure equalizing valve 105, and adjusts the oxygen-enriched gas separated and generated from the feed air to a predetermined flow rate by flow rate setting devices such as pressure adjusting valve 108 and control valve 109, and then supplies the gas to the user via cannula 111.

Air normally contains approximately 21% oxygen gas, approximately 77% nitrogen gas, 0.8% argon gas, and 1.2% carbon dioxide etc. Such a device separates out oxygen required as breathing gas. The oxygen-enriched gas is separated in the adsorption step as follows: feed air is supplied from compressor 101 as pressurized air sequentially to adsorption cylinder 102 of feed air supply target, filled with an adsorbent such as zeolite selectively adsorbing nitrogen molecules over oxygen molecules, via a switched flow path by controlling opening/closing of supply valve 103 and exhaust valve 104, and in the pressurized adsorption cylinder, approximately 77% nitrogen gas in the feed air is selectively adsorbed/removed.

The adsorption cylinder consists of a cylindrical vessel filled with an adsorbent selectively adsorbing nitrogen over oxygen. The number of adsorption cylinders is determined in relation to the amount of oxygen produced, and a two-cylinder type shown in Fig. 1 or multi-cylinder type adsorption cylinder is preferably used for continuous and efficient production of oxygen-enriched gas from feed air. The oxygen-enriched gas mainly composed of oxygen unadsorbed in the adsorption cylinder flows into product tank 107 via check valve 106 provided for prevention of backflow into the adsorption cylinder, and is temporarily stored there.

Continuous production of oxygen-enriched gas requires the nitrogen adsorbed to be desorbed/removed from the adsorbent packed in the adsorption cylinder. In the desorption step, the adsorption cylinder is connected to the exhaust line by closing the supply valve and opening the exhaust valve and is switched from a pressurized state to an atmospheric open state, and the adsorbent is regenerated by desorbing the nitrogen adsorbed under pressurization.

Two adsorption cylinders operate switching each step as staggered with each other to produce oxygen continuously: while one adsorption cylinder is at an adsorption step for producing oxygen, the other adsorption cylinder is at a desorption step for regenerating the adsorbent.

Oxygen-enriched gas is produced from feed air and temporarily stored in product tank 107. The oxygen-enriched gas stored in this product tank contains high concentration oxygen gas of, for example, 95%, and is supplied to the patient after controlling the supply flow rate and pressure by flow rate setting devices such as pressure adjusting valve 108 and control valve 109. The flow rate and oxygen concentration of the oxygen-enriched gas supplied to the user are detected by an ultrasonic oxygen concentration/flow rate sensor, which enables control of oxygen generation using feedback-control of the compressor rotation speed and the opening/closing time of the flow path switching valve based on the detected oxygen concentration value and the oxygen supply flow rate value.

The adsorbent selectively adsorbing nitrogen over oxygen to be filled in the adsorption cylinder, molecular sieve zeolites such as Na-X type, Li-X type, and MD-X type, adsorbs nitrogen and, at the same time, also adsorbs moisture in the air, which enables the oxygen-enriched gas produced to be separated as a nearly bone-dry gas. Continuous inhalation of such oxygen-enriched gas may cause drying of the nostrils etc., and for the purpose of preventing this, a humidifier may be provided in the piping that connects the product tank temporarily storing the generated oxygen-enriched gas to the cannula supplying oxygen to the patient.

Adsorption-type oxygen concentrator 100 takes in outside air through air intake port 112 (device air intake port), adsorbs and removes nitrogen from the feed air, separates and supplies oxygen, and also reduces the pressure to remove the nitrogen adsorbed to the adsorbent, which is then exhausted through exhaust port 113 (device exhaust port). In addition, components built into the device, such as compressor 101 and electronic control means for controlling the switching of the flow paths of the adsorption cylinders, generate heat, which necessitates cooling. For this reason, the oxygen concentrator is equipped with cooling fan 110, takes in outside air from air intake port 112 for air-cooling, and exhausts the exhaust heat air together with the nitrogen-rich air from exhaust port 113 to outside the system.

### [Noise reduction box]

The primary requirements for a portable oxygen concentrator are small size/light weight, and power saving. Achieving such specifications tends to facilitate reduction of structural members used for noise reduction. However, since oxygen inhalation therapy is performed not only during the day but also during sleep at night, noise reduction is also required so as not to disturb the sleep of the patient and his/her family. The present invention develops a noise reduction box as a measure to reduce noise for home use, and employs a method of storing a portable oxygen concentrator in the noise reduction box.

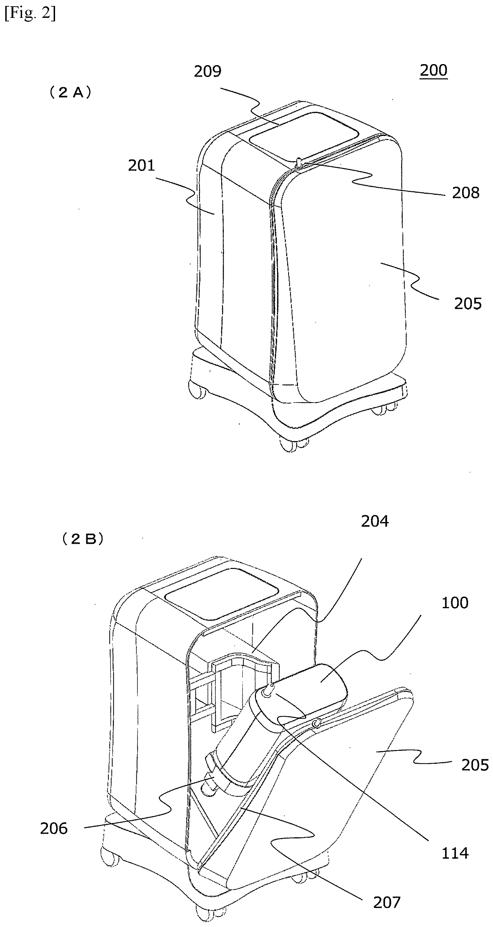

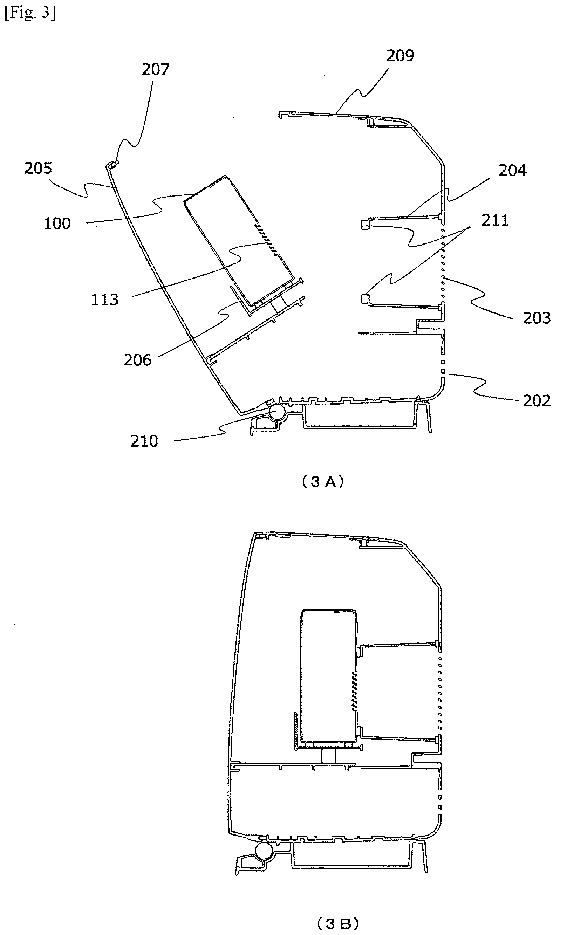

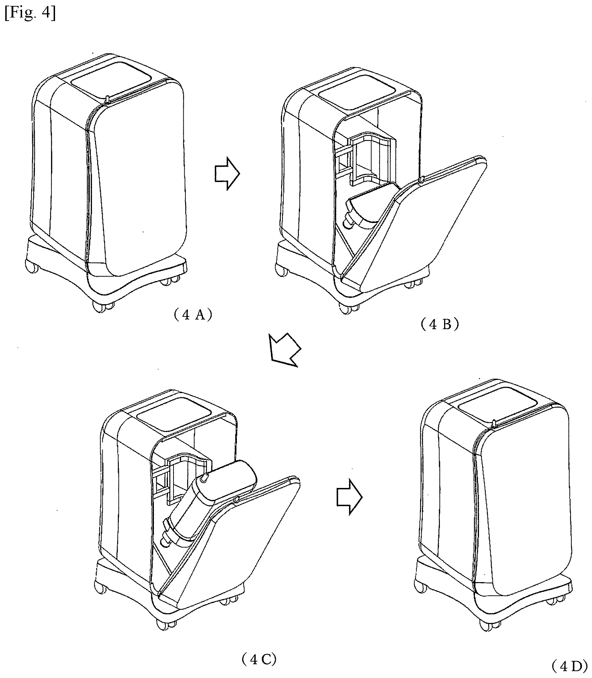

Figs. 2 to 4 show an embodiment of the noise reduction box of the present invention. Fig. 2 is a perspective view of the noise reduction box, showing a view when the opening/closing door is closed (2A), and a view when the opening/closing door is open and an oxygen concentrator is stored (2B). Fig. 3 is a cross-sectional view of the noise reduction box, showing a view when the opening/closing door is open (3A) and a view when the opening/closing door is closed (3B). Fig. 4 shows a process diagram for storing the oxygen concentrator in the noise reduction box.

Noise reduction box 200 is equipped with hollow resin housing 201, air intake port 202 (box air intake port) for taking air into the noise reduction box, and exhaust port 203 (box exhaust port) for exhausting air including exhaust heat air outside the system, and exhaust duct 204 is directly connected to exhaust port 203. The side of the noise reduction box is opening/closing door 205, and adopted is a mechanism in which rotating shaft 210 is provided at the lower part of the side, and the opening/closing door is opened diagonally upward by pulling the top of the door toward an operator with the rotating shaft at the lower part of the door as a fulcrum instead of a method such as a single swing side door and a top face opening.

Such opening/closing door 205 is provided inside with storage portion 206 for storing portable oxygen concentrator 100. Such a structure allows one hand to open the door of the noise reduction box and the other hand to hold and slide oxygen concentrator 100 into the storage portion of the door, which enables the oxygen concentrator to be easily stored.

The position of storage portion 206 provided inside opening/closing door 205 is determined so that the position of exhaust port 113 of the device to be stored coincides with the position of exhaust duct 204. Closing opening/closing door 205 allows the exhaust port of the stored oxygen concentrator to come into close contact and be connected with the inlet of the exhaust duct of the noise reduction box, and thus allows the exhaust air from the oxygen concentrator to be exhausted directly outside the system from the box exhaust port without circulating inside the noise reduction box.

When the storage portion of the device is provided on the opening/closing door, the center of gravity is on the door side, and thus storing the device with the opening/closing door diagonally open makes the noise reduction box likely to fall down. Although not shown in Figs., the lower part of the noise reduction box of the present invention is equipped with a weight to prevent falling down of the noise reduction box against the center of gravity position change when storing the device inside.

Power saving is also one of important requirement specifications of the oxygen concentrator. The requirement specification influences a battery life of portable devices during outdoor use. Although the noise reduction box is used at home and thus the power source is available, a structure of the noise reduction box requiring additional electricity, such as providing a cooling mechanism, should be desirably avoided due to electricity cost reduction. Providing exhaust duct 204 connected directly to exhaust port 113 of oxygen concentrator 100 allows the blowing capacity of cooling fan 110 of the oxygen concentrator to draw outside air into the noise reduction box and to exhaust air from the noise reduction box.

In order to enhance the noise reduction effect, exhaust duct 204 may be provided with a sound absorbing material attached to the inner surface or may have a multistage folded structure. However, since the sound-absorbing material has a heat insulation effect and the multistage folded structure causes exhaust resistance, a straight exhaust duct without providing any special structure is preferably used to exhaust the air directly from exhaust port 203 in order to exhaust the exhaust heat air outside the system by only the cooling fan incorporated in the oxygen concentrator.

A noise reduction effect can also be achieved by attaching sound-absorbing material to the inside of the noise reduction box housing. However, such sound absorbing material is preferably not provided inside in order to exhibit the exhaust heat effect in the box.

A closed-cell foam material is used as sealant 207 between the opening/closing door and the housing. Preferably, a urethane foam material having a predetermined thickness is used. Having low-resilience cushioning performance allows the exhaust port of the oxygen concentrator to come into close contact with the exhaust duct at the same time as closing the door, and thus exhaust leakage from the connection portion to be prevented. The same foam material is preferably provided at the inlet of the exhaust duct as foam material 211 to ensure close contact.

The primary purpose of the noise reduction box is to prevent sound leakage, and thus requires openings to be a minimum. Required to be a minimum are openings, such as air intake port 202 for taking in feed air necessary for oxygen generation and cooling air, exhaust port 203 for exhausting exhaust heat air containing nitrogen, and an opening for an alarm sound. In contrast, the oxygen produced by the oxygen concentrator needs to be taken out from the noise reduction box. In the present invention, oxygen supply connection portion 208 is provided on the housing of the noise reduction box, and oxygen supply end 114 of the adsorption-type oxygen concentrator is connected to oxygen supply connection portion 208 with a relay tube, and thus oxygen can be taken from the oxygen concentrator stored in the noise reduction box to the outside of the box. Further, nasal cannula 111 connected to oxygen supply connection portion 208 allows oxygen to be supplied to the user.

Although the noise reduction box is designed on the assumption of being used in a specified manner by the patient, the patient will not necessarily follow this. Using a closed-cell foam material of a predetermined thickness for sealant 207 between the opening/closing door and the housing enables a user, when the user returns home using a portable oxygen concentrator connected with nasal cannula 111, to store the device in the noise reduction box and draw out the nasal cannula through the sealed part of the opening/closing door while keeping the nasal cannula connected and maintaining sealability for preventing sound leakage. For this purpose, the sealant is provided preferably with a thickness larger than the diameter of the cannula. For example, when a cannula having an outer diameter of 7 mm is used, low-resilience urethane foam having a thickness of 10 mm can be used for sealant 207. This allows the opening/closing door of the noise reduction box to be closed and oxygen to be supplied through the cannula without crushing the cannula or causing sound leakage or air leakage.

The top face of the housing of the noise reduction box is equipped with window 209 made of a transparent resin material such as acryl or polycarbonate for the display unit of the oxygen concentrator to be seen from the outside. Although extracting the device signal from the stored oxygen concentrator and providing a separate display unit on the noise reduction box greatly improves the visibility of the device's operating status from outside, this would result in the labor of connecting the signals, increased manufacturing costs, and increased power consumption, and thus the operating status is preferably to be directly viewed with the naked eye through the transparent window.

### [Industrial Applicability]

The noise reduction box of the present invention can provide equipment that can facilitate noise reduction of a device, such as an oxygen concentrator, which generates an annoying noise when used at home at night, by storing the device as a whole while solving the problems of power saving and exhaust heat. In addition, use of a small and lightweight medical device that can be usually carried with enables patients to expand their field of activities and improve their QOL.

### [Reference Signs List]

100 Adsorption-type oxygen concentrator
101 Compressor
102 Adsorption cylinder
103 Supply valve
104 Exhaust valve
105 Pressure equalizing valve
106 Check valve
107 Product tank
108 Pressure Regulating valve
109 Control valve
110 Cooling fan
111 Cannula (nasal cannula)
112 Air intake port (device air intake port)
113 Exhaust port (device exhaust port)
114 Oxygen supply end
200 Noise reduction box
201 Housing
202 Air intake port (box air intake port)
203 Exhaust port (box exhaust port)
204 Exhaust duct
205 Opening/closing door
206 Storage portion
207 Sealant
208 Oxygen supply connection portion
209 Window
210 Rotating shaft
211 Foam material

## Claims

1. A noise reduction box equipped with a storage portion for storing a noise-generating device inside, comprising:
a housing of the noise reduction box; and on the housing, an opening/closing door, a box air intake port for taking outside air into the noise reduction box, and a box exhaust port for exhausting air in the noise reduction box; and inside the box, an exhaust duct for guiding exhaust air to the box exhaust port;
wherein a device exhaust port of the device stored in the storage portion is in close contact with an inlet of the exhaust duct when the opening/closing door is closed.

2. The noise reduction box according to claim 1, wherein the storage portion is fastened to the opening/closing door, and the device stored in the storage portion moves to a predetermined position in conjunction with the action of closing the opening/closing door, and the device exhaust port comes into close contact with the inlet of the exhaust duct.

3. The noise reduction box according to claim 2, wherein the opening/closing door is provided at the side of the noise reduction box, is connected to a box body via a rotating shaft arranged horizontally under the noise reduction box and when pulled toward an operator at the upper part, opens diagonally upward with the rotating shaft as a fulcrum, and the device can be stored in the storage portion from the diagonally upward opening.

4. The noise reduction box according to claim 3, wherein the device further has a cooling fan, and by using the cooling fan, draws cooling air from the outside air into the noise reduction box to cool inside the noise reduction box while cooling inside the device by the cooling air taken through the device air intake port and releases the air outside via the exhaust duct from the device exhaust port.

5. The noise reduction box according to claim 4, further comprising a sealant made of a closed-cell foam material between the opening/closing door and the housing of the noise reduction box.

6. The noise reduction box according to claim 5, wherein the device is an adsorption-type oxygen concentrator.

7. The noise reduction box according to claim 6, further comprising an oxygen supply connection portion on the housing of the noise reduction box, and a relay tube for connecting an oxygen supply end of the adsorption-type oxygen concentrator to the oxygen supply connection portion.

8. An oxygen supply system comprising: an adsorption-type oxygen concentrator, and a noise reduction box equipped with a storage portion for storing the adsorption-type oxygen concentrator inside;
a housing of the noise reduction box; and on the housing, an opening/closing door, a box air intake port for taking outside air into the noise reduction box, and a box exhaust port for exhausting air in the noise reduction box; and inside the box, an exhaust duct for guiding exhaust air to the box exhaust port;
wherein an exhaust port of the adsorption-type oxygen concentrator stored in the storage portion is in close contact with an inlet of the exhaust duct when the opening/closing door is closed.

9. The oxygen supply system according to claim 8, wherein the opening/closing door is provided at the side of the noise reduction box, is connected to a box body via a rotating shaft arranged horizontally under the noise reduction box and when pulled toward an operator at the upper part, opens diagonally upward with the rotating shaft as a fulcrum, and when the opening/closing door is closed, the exhaust port of the adsorption-type oxygen concentrator stored in the storage portion from the diagonally upward opening comes into close contact with the inlet of the exhaust duct.

10. The oxygen supply system according to claim 9, further comprising an oxygen supply connection portion on the housing of the noise reduction box, and a relay tube for connecting an oxygen supply end of the adsorption-type oxygen concentrator to the oxygen supply connection portion.
